# EUROPEAN PATENT APPLICATION

(11) **EP 4 353 248 A1**
(43) Date of publication of application: **17.04.2024**
(21) Application number: 22819555.8
(22) Date of filing: 08.06.2022
(51) Int. Cl.: A61K 38/08, A61P 3/14, A61P 5/20

(54) **CALCIUM-SENSING RECEPTOR AGONIST COMPOSITION AND APPLICATION THEREOF**

(30) Priority: 08.06.2021 CN 202110637307
(71) Applicant: Beijing Tuo Jie Biopharmaceutical Co. Ltd., Beijing 102206 (CN)
(72) Inventor: TONG, Xinyong, Beijing 102206 (CN); ZOU, Aifeng, Beijing 102206 (CN); WANG, Haifeng, Beijing 102206 (CN); YANG, Tingting, Beijing 102206 (CN); YANG, Yi, Beijing 102206 (CN); ZHOU, Yin, Beijing 102206 (CN)
(74) Representative: Regimbeau
(86) International application number: PCT/CN2022/097594
(87) International publication number: WO 2022/257958

(57) **Abstract**

Provided are a calcium-sensing receptor (CaSR) agonist composition and an application thereof. Specifically, provided is a pharmaceutical composition of a polypeptide CaSR agonist, the composition comprising an active ingredient, a buffer and an osmotic pressure regulator. The present composition has high stability, may be stored for a long time, may effectively reduce the plasma parathyroid hormone, and has a low level of histamine release.

## Description

### TECHNICAL FIELD

The present disclosure belongs to the technical field of pharmaceuticals, and relates to a composition of a calcium-sensing receptor agonist suitable for injection and use thereof.

### BACKGROUND

Calcium-sensing receptor (CaSR) refers to a G-protein coupled receptor (GPCR) family A member distributed on the cell surface in human parathyroid glands. Secretion of parathyroid hormone is highly regulated by calcium-sensing receptor on parathyroid cell surface to maintain the steady levels of minerals in human body. The calcium-sensing receptor continuously monitors subtle changes in calcium ion concentration in human body and responds accordingly by altering the level of parathyroid hormone secretion.

In patients with chronic kidney disease, the need of steady levels of calcium and phosphorus ions in the body results in the continuous secretion of parathyroid hormone in the parathyroid glands. The continuous secretion of parathyroid hormone is initially adaptive, but eventually leads to hyperplasia of the parathyroid glands and excessive levels of parathyroid hormone in the body and induces the formation of secondary hyperparathyroidism as chronic kidney disease progresses. Studies have shown that persistent secondary hyperparathyroidism will lead to the loss of calcium-sensing receptor and vitamin D receptor on parathyroid cell surface. These downstream pathological effects caused by the disease further lead to dysregulation of the steady mineral levels by the parathyroid glands.

Calcimimetics generally refer to compounds that have similar physiological functions and mechanisms of action to calcium ion and can directly activate calcium-sensing receptor on parathyroid cell surface. Cinacalcet hydrochloride is an organic small-molecule calcimimetic developed by Amgen, which can activate the calcium-sensing receptor on the surface of a parathyroid organ and inhibit the secretion level of parathyroid hormone so as to achieve the purpose of treating related metabolic diseases such as secondary hyperparathyroidism. Cinacalcet hydrochloride has been approved for the treatment of secondary hyperparathyroidism in chronic kidney disease patients receiving dialysis, and is administered orally one to two times daily with a dose up to 90 mg. Cinacalcet hydrochloride shows clinically excellent efficacy in reducing plasma parathyroid hormone levels in patients with secondary hyperparathyroidism. However, significant drug-induced side effects such as nausea, vomiting and diarrhea associated with gastrointestinal side effects are observed during patient use. In addition, the oral administration of cinacalcet hydrochloride imposes great burden in chronic kidney disease patients receiving dialysis, and cinacalcet hydrochloride has been demonstrated to inhibit cytochrome 450 and induce associated drug-drug interaction. Such adverse effects associated with the use of cinacalcet hydrochloride reduce patient adherence and compliance to some extent.

Therefore, there is an urgent clinical need for a calcium-sensing receptor agonist compound that can be intravenously administered and can reduce the secretion of parathyroid hormone by activating the calcium-sensing receptor on parathyroid cell surface to achieve the therapeutic purpose of treating associated metabolic diseases such as secondary hyperparathyroidism. Such calcium-sensing receptor agonist compounds can significantly improve the treatment adherence and compliance in patients with chronic kidney disease.

WO2021115272 describes a series of polypeptide calcium-sensing receptor agonist compounds, wherein the compound of formula (I) has agonist effects on human calcium-sensing receptors to reduce plasma parathyroid hormone and serum calcium ion levels, and can be used for the treatment of metabolic diseases such as secondary hyperparathyroidism, tumor-induced hypercalcemia, etc.

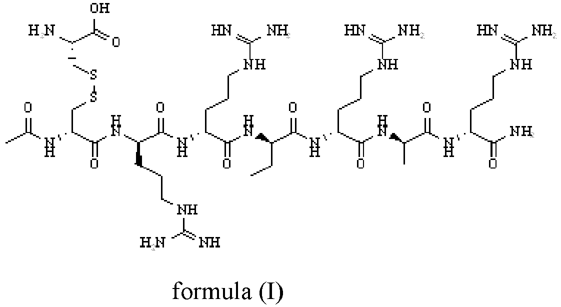

### SUMMARY

The present disclosure provides a pharmaceutical composition of a calcium-sensing receptor agonist and use thereof. The pharmaceutical composition provided by the present disclosure has high stability, can be stored for a long time, can effectively reduce the plasma parathyroid hormone, and has low histamine release level and small side effects.

The pharmaceutical composition provided by the present disclosure has low sensitivity and good safety, and does not cause *in vivo* hemolysis.

The pharmaceutical composition provided by the present disclosure comprises an active ingredient, a buffer and an osmotic pressure regulator, wherein the active ingredient is a compound of formula (I) or a pharmaceutically acceptable salt thereof:

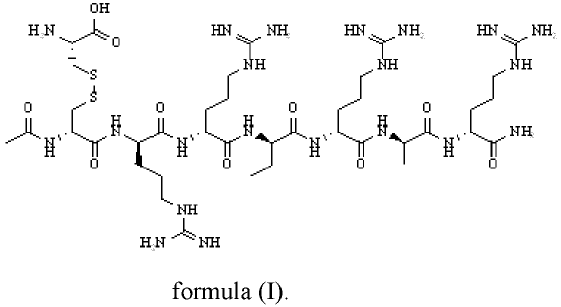

The amino acid sequences in the present disclosure are represented by the standard single-letter or three-letter codes for amino acids, i.e., alanine (Ala, A), cysteine (Cys, C), arginine (Arg, R) and *D*-2-aminobutyric acid (*D*-Abu). The compound of formula (I) of the present disclosure can also be represented by the following sequence: Ac-c(C)-r-r-(*D*-Abu)-r-a-r-NH₂.

The pharmaceutical composition provided by the present disclosure has a pH value of 2.0-3.5. In some embodiments, the composition has a pH value of 3.0-3.5. In some embodiments, the composition has a pH value of 3.3.

In the pharmaceutical composition provided by the present disclosure, the active ingredient is a hydrochloride of the compound of formula (I).

In certain embodiments, the number of hydrochloric acid bound to the compound of formula (I) in the hydrochloride of the compound of formula (I) may be 1-10 (may be any number between 1 and 10, i.e., an average); preferably, the number of hydrochloric acid is 4-8, more preferably 4-5; optional numbers of hydrochloric acid include, but are not limited to, 1, 1.5, 2, 2.5, 3, 3.5, 4, 4.5, 5, 5.5, 6, 6.5, 7, 7.5, 8, 8.5, 9, 9.5 and 10.

In the pharmaceutical composition provided by the present disclosure, the content of the active ingredient based on free base is 1-10 mg/mL. In some embodiments, the content of the active ingredient based on free base is 2.5-10 mg/mL. In some embodiments, the content of the active ingredient based on free base is 5 mg/mL. The content of the active ingredient based on free base may be 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL and 10 mg/mL.

In the pharmaceutical composition provided by the present disclosure, the buffer is selected from the group consisting of one or more of succinate, citrate and phosphate. In some embodiments, the buffer is succinate.

In the pharmaceutical composition provided by the present disclosure, the osmotic pressure regulator is selected from the group consisting of one or more of sodium chloride and glucose. In some embodiments, the osmotic pressure regulator is sodium chloride.

In some embodiments, the pharmaceutical composition provided by the present disclosure further comprise a pH adjuster, wherein the pH adjuster is one or more of sodium hydroxide and hydrochloric acid.

In the pharmaceutical composition provided by the present disclosure, the buffer has a mass/volume percentage of 0.05%-1%. In some embodiments, the buffer has a mass/volume percentage of 0.1%-0.5%. In some embodiments, the buffer has a mass/volume percentage of 0.118%. Optional mass/volume percentages are 0.05%, 0.06%, 0.07%, 0.08%, 0.09%, 0.10%, 0.11%, 0.115%, 0.118%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.35%, 0.40%, 0.45% and 0.50%.

In the pharmaceutical composition provided by the present disclosure, the osmotic pressure regulator has a mass/volume percentage of 0.5%-2%. In some embodiments, the osmotic pressure regulator has a mass/volume percentage of 0.5%-1%. In some embodiments, the osmotic pressure regulator has a mass/volume percentage of 0.85%. Optional mass/volume percentages are 0.5%, 0.55%, 0.6%, 0.65%, 0.7%, 0.75%, 0.8%, 0.85%, 0.9%, 0.95% and 1%.

The present disclosure provides a pharmaceutical composition comprising 2.5-10 mg/mL of an active ingredient based on free base, 0.1%-0.5% (w/v) of a buffer and 0.5%-1% (w/v) of an osmotic pressure regulator, wherein the active ingredient is a compound of formula (I) or a pharmaceutically acceptable salt thereof. In some embodiments, the active ingredient is a hydrochloride of the compound of formula (I):

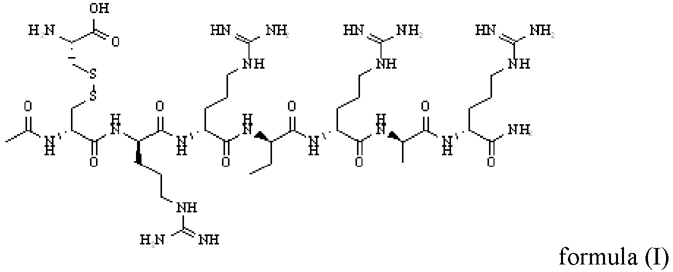

The buffer is one or more of succinate, citrate and phosphate. In some embodiments, the buffer is succinate; the osmotic pressure regulator is one or more of sodium chloride and glucose. In some embodiments, the buffer is succinate sodium chloride.

In the pharmaceutical composition provided by the present disclosure, the generated total impurity content is 10% or less when the pharmaceutical composition is stored at a temperature of 2-8 °C for 3 months. In some embodiments, the generated total impurity content is 8% or less when the pharmaceutical composition is stored at a temperature of 2-8 °C for 3 months.

In some embodiments, the pharmaceutical composition provided by the present disclosure is suitable for injection.

The present disclosure provides use of the composition described above in preparing a medicament for reducing parathyroid hormone levels in a subject or for treating secondary hyperparathyroidism or tumor-induced hypercalcemia. In some embodiments, the hyperparathyroidism is secondary hyperparathyroidism in a subject suffering from a chronic kidney disease.

Another aspect of the present disclosure provides a method for preparing the pharmaceutical composition described above, comprising mixing a formula amount of a buffer, sodium chloride and water for injection, stirring until the mixture is completely dissolved, mixing a formula amount of the compound of formula (I) with the solution described above, stirring until the mixture is completely dissolved; adjusting the pH to a target pH value with sodium hydroxide, adding water for injection to the full amount, stirring to mix well, sterilizing, filtering and bottling.

In the specification and claims of the present disclosure, unless otherwise specified, the scientific and technological terms used herein have meanings generally understood by those skilled in the art. However, definitions and explanations for some of the related terms are provided below for a better understanding of the present disclosure.

As used herein, "mass/volume percentage" (w/v) refers to the mass (g) of an ingredient contained in per 100 mL of a liquid system, i.e., g/100 mL.

WO2021115272 is incorporated herein by reference in its entirety.

### BRIEF DESCRIPTION OF THE DRAWINGS

FIG. 1 shows the hemolytic effect of the compound of formula (I) against human red blood cells *in vitro,* wherein * denotes positive control (polyethylene glycol octyl phenyl ether), and # denotes PBS buffer.
FIG. 2 shows the effect of the compound of formula (I) in reducing parathyroid hormone levels in normal rats *in vivo.*
FIG. 3 shows the effect of the compound of formula (I) in reducing serum calcium ion levels in normal rats *in vivo.*

### DETAILED DESCRIPTION

Hereinafter, the present disclosure will be explained in more details with reference to the examples. The examples are only used to illustrate the technical solutions of the present disclosure, rather than limit the essence and scope of the present disclosure. All the pharmaceutical excipients used in the present disclosure are commercially available.

### 1. Experimental reagent

| No. | Reagent | Source |
|---|---|---|
| 1 | Rink-amide MBHA resin | Sunresin, Xi'an |
| 2 | HCTU | Highfine, Suzhou |
| 3 | 4-methylmorpholine | TCI Chemicals |
| 4 | Acetonitrile (chromatographic grade) | Sigma-Aldrich |
| 5 | *N,N-*dimethylformamide | SinoPharm |
| 6 | Dichloromethane | SinoPharm |
| 7 | Trifluoroacetic acid | TCI Chemicals |
| 8 | Triisopropylsilane | TCI Chemicals |
| 9 | Methyl *tert*-butyl ether | TCI Chemicals |
| 10 | 4-methylpiperidine | TCI Chemicals |
| 11 | L-cysteine | Sigma-Aldrich |
| 12 | Fmoc-D-Cys(Trt)-OH | GL Biochem |
| 13 | Fmoc-D-Arg(Pbf)-OH | GL Biochem |
| 14 | Fmoc-D-Ala-OH | GL Biochem |
| 15 | Fmoc-D-Abu-OH | GL Biochem |
| 16 | 2,2'-Dipyridyldisulfide | GL Biochem |

### 2. Experimental instruments

| No. | Instrument | Source |
|---|---|---|
| 1 | Prelude-X multichannel polypeptide synthesizer | Protein Technology |
| 2 | H-CLASS analytical ultra performance liquid chromatograph | Waters |
| 3 | Xevo liquid chromatography/mass spectrometry | Waters |
| 4 | Labconco multifunctional freeze dryer | Thermo-Fisher Scientific |
| 5 | Prep150 preparative high performance liquid chromatograph | Waters |
| 6 | Multichannel high-speed centrifuge | Sigma |

Solid phase peptide synthesis was performed on a Prelude-X automatic polypeptide synthesizer using the Fmoc/tBu synthesis strategy starting from Rink-amide MBHA resin (0.1 mmol). Coupling was performed using 10 equivalents of amino acid residues activated with HCTU and 4-methylmorpholine (the molar ratio of HCTU:4-methylmorpholine:amino acid residues was 1:2:1) in N,N-dimethylformamide at room temperature for 25 min.

After the completion of the above peptide-resin synthesis, in a solution containing 90:5:5 (v/v/v) of trifluoroacetic acid:triisopropylsilane:water and 2,2'-dipyridyldisulfide (1 mmole), cleavage of the polypeptide from the solid phase resin, removal of the side chain protecting group and activation of the D-Cys side chain sulfhydryl were simultaneously accomplished at room temperature within 2 h. After the reaction was completed, the mixture was filtered and the resin was washed for 2 times by using trifluoroacetic acid. The filtrates were combined before a large amount of frozen methyl tert-butyl ether was added to precipitate a solid. The mixture was centrifuged and the supernatant was discarded to obtain a crude product of the polypeptide, which was then dried and weighed.

The crude polypeptide obtained above and L-Cys (0.1 mmol) were dissolved in PBS buffer (pH = 7.4) and reacted with shaking at room temperature. The production of compound **1** was monitored by ultra-performance liquid chromatography. After completion of the reaction, trifluoroacetic acid (300 µL) was added to the mixture to quench the reaction and for subsequent purification.

The mixture obtained above was filtered through a 0.22 µm membrane and separated by a Waters Prep150 preparative reverse-phase high performance liquid chromatography system with buffers A (0.1% trifluoroacetic acid, aqueous solution) and B (0.1% trifluoroacetic acid, 90% acetonitrile, aqueous solution). The preparative chromatographic column was an X-SELECT OBD C-18 (Waters) reversed-phase chromatographic column, the detection wavelength of a chromatograph was set as 220 nm in the purification process, and the flow rate was 15 mL/min. The purified polypeptide product of compound 1 was obtained after the relevant fractions were collected and lyophilized (45% yield). The purity and the compound identity of the pure polypeptide product were determined by analytical ultra-performance liquid chromatography and ultra-performance liquid chromatography/mass spectrometry, wherein the purity of the compound was 96.78%, and the molecular weight of the compound was 1109.60 Da.

| Compound No. | Sequence |
|---|---|
| 1 | Ac-c(C)-(D-Phg)-r-r-r-a-r-NH₂ |

"Ac-c(C)" denotes that an acetylated cysteine in D configuration (c) at the amino terminus is linked to another cysteine in L configuration (C) by a disulfide bond; "r-NH₂" denotes an amidated arginine in the D configuration (r) at the carboxyl terminus.

### Example 2

The compound of formula (I) was synthesized using synthetic protocols similar to that of Example **1,** and the purity and molecular weight of the synthesized polypeptides were determined by analytical ultra-performance liquid chromatography and ultra-performance liquid chromatography/mass spectrometry, wherein the purity of the compound of formula (I) was 95.79%, and the molecular weight of the compound was 1062.29 Da.

### Example 3

### Preparation of Hydrochloride of Compound of Formula (I)

1. Rink Amide-AM resin (1034.4 g) was taken and added to a glass reactor, and DMF was added to swell the resin. A 20% PIP/DMF (V/V) solution was added for reaction to remove Fmoc. The resin was washed with DMF and turned blue in a ninhydrin test. Fmoc-D-Arg(Pbf)-OH (1167.6 g) and Oxyma (383.6 g) were weighed and dissolved in DMF (5.0 L) and DCM (5.0 L), and then DIC (340.5 g) was added. The mixture was well stirred and added to a glass reactor for reaction. The reaction end point was detected using ninhydrin (stopping the reaction if the resin was colorless and transparent, and prolonging the reaction time if the resin was colored, the same applies below). After the reaction was completed, the resin was washed with DMF, IPA, DMF, IPA and DMF sequentially. AczO (368.1 g) and DIEA (467.4 g) were weighed, and DMF (5.0 L) and DCM (5.0 L) were added. The mixture was well stirred and added to a glass reactor for reaction. A small amount of the resin was detected using ninhydrin until the reaction end point. After the reaction was completed, the resin was washed with DMF, IPA, DMF, IPA and DMF sequentially.
2. The subsequent amino acids were coupled sequentially according to the same coupling method to obtain 2.5 kg of N-Ac-D-Cys(Trt)-D-Arg(Pbf)-D-Arg(Pbf)-D-Abu-D-Arg(Pbf)-D-Ala-D-Arg(Pbf)-Rink amide resin.
3. TFA/TIS/H2O (V:V:V = 97:2.5:0.5) lysis buffer was added to a 50 L reactor, DPDS (793.2 g) was added, and the mixture was stirred and dissolved. The product of 2 (2.5 kg) was added to the reactor, and the mixture was stirred at room temperature for reaction and filtered. The filtrate was added to IPE/ACN (V:V = 7:1), and 850 g of the precursor polypeptide was collected (75.4% yield).
4. 6.73 L of water was added to a 20 L reactor, and H-L-Cys-OH.HCl.H₂O (118.4 g) was added. After the mixture was stirred and dissolved, the precursor polypeptide (850 g) of 3 was added for reaction. After the reaction was completed, the mixture was slowly flushed into a 0.01 moL/L HCl/IPA solution, stirred and centrifuged. The filter cake was collected. The filter cake was dried to obtain 700 g of the crude target polypeptide (79% yield).
5. 700 g of the crude peptide of 4 was purified by using a Hanbon purification system at a wavelength of 254 nm using a packing material of C18 and a mobile phase of 0.1% TFA/H₂O and 0.1% TFA/acetonitrile. Fractions of the target peak were collected to obtain 90 L of a purified liquid with a process yield of 64.1%.
6. A nanofiltration system was used, and a hydrochloric acid solution was continuously added while trifluoroacetate was removed from the sample solution to obtain a hydrochloride solution of the compound of formula (I). After concentration and lyophilization, 240 g of the hydrochloride of the compound of formula (I) was obtained with a yield of 90%, a mass spectrometry signal of 1061.5367 Da (theoretical value of 1061.5447 Da), an HPLC purity of 98.33% and an overall yield of 34.4%.

According to potentiometric titration of General Chapter 0701, Chinese Pharmacopoeia, Volume IV, 2020 Edition, the number of hydrochloric acid bound to the compound of formula (I) was 4.4.

### Example 4

### Preparation Process for Pharmaceutical Composition

| Components | Amount | Ratio (%, W/V) | Effect |
|---|---|---|---|
| Hydrochloride of compound of formula (I) | 5.0 mg (calculated based on free base) | 0.50% (calculated based on free base) | Active substance |
| Succinic acid | 1.18mg | 0.118% | Buffer |
| Sodium chloride | 8.50mg | 0.85% | Osmotic pressure regulator |
| Sodium hydroxide | An appropriate amount | N/A | pH value regulator |
| Water for injection | To 1 mL | N/A | Solvent |

According to the formulation in the above table, about 70% of the total volume of water for injection was taken, the formula amount of buffer and sodium chloride were added, and the mixture was stirred until visually completely dissolved. Then the formula amount of a hydrochloride of the compound of formula (I) was added. The mixture was stirred until completely dissolved and was adjusted to pH 3.3 ± 0.10 with 1 mol/L sodium hydroxide. Water for injection was added to the full amount. The mixture was stirred to mix well. After sterilization and filtration, the mixture was bottled, and the bottles were stoppered.

### Example 5

### Composition pH Value Screening

The pH value screening experiment was carried out according to the formulation of Table 3. When in preparation, the pH value was not adjusted, and the pH value of the formulation solution was about 2.7. The pH value was adjusted to 2.0, 3.0, 3.3, 3.5, 4.0, 4.5, 5.0 and 6.0 with 1 M sodium hydroxide or 1 M hydrochloric acid, respectively. The stability of the composition at 2-8 °C and 25 °C was investigated, and the experimental results are shown in Table 4.

**Table 3. Formulation of composition pH value screening**

| Components | Amount |
|---|---|
| Compound of formula (I) | 5 mg/mL (calculated based on free base) |
| Succinic acid | 10mM |
| Sodium chloride | 0.85% |
| Sodium hydroxide/hydrochloric acid | An appropriate amount |
| pH value | 2.0, 3.0, 3.3, 3.5, 4.0, 4.5, 5.0, 6.0 |

| | |
|---|---|
| Note: the content of API is 95.6%, and the total impurities are 3.9% | |

**Table 4. pH value screening list**

| Test items | pH value | 0d | 25°C | | | 2-8°C | | |
|---|---|---|---|---|---|---|---|---|
| | | | 7d | 14d | 1M | 1M | 2M | 3M |
| Total impurity% | 2.0 | 4.11 | 5.23 | 7.14 | 8.73 | 4.79 | 5.08 | 5.93 |
| | 3.0 | 4.07 | 4.50 | 5.04 | 5.38 | 4.18 | 4.16 | 4.53 |
| | 3.3 | 4.15 | 4.51 | 5.16 | 5.48 | 4.14 | 4.21 | 4.36 |
| | 3.5 | 4.15 | 4.20 | 5.07 | 5.90 | 4.37 | 4.42 | 4.54 |
| | 4.0 | 4.41 | 4.61 | 6.40 | 6.70 | 4.76 | 4.81 | 5.21 |
| | 4.5 | 4.26 | 7.00 | 7.29 | 7.51 | 5.60 | 5.76 | 5.91 |
| | 5.0. | 5.71 | 8.00 | 8.66 | 9.05 | 6.74 | 6.76 | 6.79 |
| | 6.0 | 8.76 | 12.22 | 16.90 | 19.15 | 9.93 | / | / |
| Content% | 2.0 | 100.3 | 99.0 | 99.0 | 93.3 | 97.2 | 96.4 | 96.2 |
| | 3.0 | 101.0 | 101.4 | 99.6 | 97.9 | 98.6 | 98.2 | 98.0 |
| | 3.3 | 100.1 | 101.0 | 99.1 | 97.1 | 98.3 | 97.7 | 98.2 |
| | 3.5 | 100.6 | 100.8 | 101.2 | 97.9 | 98.3 | 98.1 | 98.6 |
| | 4.0 | 100.2 | 100.1 | 99.0 | 96.7 | 98.0 | 97.5 | 97.8 |
| | 4.5 | 99.5 | 97.6 | 98.0 | 95.7 | 97.9 | 96.5 | 95.7 |
| | 5.0. | 100.0 | 98.1 | 96.2 | 94.2 | 95.1 | 95.1 | 95.6 |
| | 6.0 | 96.6 | 93.8 | 88.3 | 85.6 | 92.5 | / | / |

The results show that: when the pH value is higher than 3.5, the content of the active ingredient is significantly reduced, and the total impurities are significantly increased. When the pH value is between 3.0 and 3.5 and the composition is placed at 2-8 °C for 3 M and 25 °C for 1 M, the content and total impurities are relatively stable, and the formulation stability is relatively good, therefore the pH value range of the pharmaceutical composition is preferably 3.0-3.5.

### Example 6

### Buffer System Screening

The stability of the composition under different buffer systems was investigated in the experiment. The screening formulation is shown in Table 5, and the experimental results are shown in Table 6.

**Table 5. Formulation of composition buffer system screening**

| Formulation/amount | 1 | 2 | 3 |
|---|---|---|---|
| Compound of formula (I) | 5 mg/mL (calculated based on free base) | | |
| Buffer salt system | Succinic acid-sodium hydroxide | Citric acid-sodium citrate | Phosphate |
| Buffer salt concentration | 10mM, 20mM, 30mM | 10mM | 20mM |
| pH value | 3.3 | | |

**Table 6. Composition buffer system screening list**

| Test items | Buffer system | 0d | 25°C | | | 2-8°C | | |
|---|---|---|---|---|---|---|---|---|
| | | | 7d | 14d | 1M | 1M | 2M | 3M |
| Total impurity% | Succinate | 4.15 | 4.51 | 5.16 | 5.48 | 4.14 | 4.21 | 4.36 |
| | Citrate | 4.02 | 4.80 | 5.68 | 5.85 | 4.21 | 4.42 | 4.52 |
| | Phosphate | 3.95 | 4.05 | 5.11 | 5.64 | 4.22 | 4.21 | 4.32 |
| Content% | Succinate | 100.1 | 101.0 | 99.1 | 97.1 | 98.3 | 97.7 | 98.2 |
| | Citrate | 101.2 | 101.2 | 99.9 | 97.3 | 99.8 | 97.6 | 99.3 |
| | Phosphate | 98.8 | 98.6 | 96.3 | 94.9 | 95.5 | 95.3 | 94.0 |
| pH value | Succinate | 3.29 | 3.37 | 3.30 | 3.32 | 3.33 | 3.29 | 3.32 |
| | Citrate | 3.30 | 3.39 | 3.33 | 3.36 | 3.37 | 3.33 | 3.35 |
| | Phosphate | 3.34 | 3.45 | 3.39 | 3.41 | 3.42 | 3.39 | 3.42 |

It can be seen from the experimental results that the 3 buffer salts and the three buffer systems of the compound of formula (I) all have good stability. Under the conditions of 25 °C for 1 M and 2-8 °C for 3 M, the succinate buffer system has minimum total impurity increase, and the content is stable.

### Example 7

The composition prepared in Example 4 was administered to beagle dogs for *in vivo* hemolysis studies. Beagle dogs were given 5 mg/mL of the pharmaceutical composition by intravenous bolus injection and observed for 4 consecutive days after a single administration. After administration, no abnormal change related to the test sample was seen in the animal general state observation, hematology related to hemolysis, blood biochemistry, urine index and blood cell smear.

The above results show that 5 mg/mL of the pharmaceutical composition of the compound of formula (I) does not cause hemolysis in the animal.

### Example 8

The present disclosure is further described and explained below with reference to specific examples in the present disclosure, but these examples are not intended to limit the scope of the present disclosure.

### 1. Reagent for in vitro and in vivo biological evaluation

| No. | Reagent | Source |
|---|---|---|
| 1 | FBS, 500 ml | ThermoFisher Scientific |
| 2 | DMEM, High Glucose, GlutaMAX, 500 ml | ThermoFisher Scientific |
| 3 | Penicillin-Streptomycin, Liquid, 100 ml (100X) | ThermoFisher Scientific |
| 4 | 1 X PBS pH 7.2-7.4 (500 ml) | Solarbio |
| 5 | 1X TrypLE Express Enzyme, no phenol red (500 ml) | ThermoFisher Scientific |
| 6 | Hygromycin B Gold solution (5 g, 1 x 50 ml, 100 mg/ml) | Invivogen |
| 7 | HEPES, 1 M | Gibco |
| 8 | MgCl₂, 1 M | Sigma-Aldrich |
| 9 | KCl, 1M | Sigma-Aldrich |
| 10 | NaCl, 5 M | Sigma-Aldrich |
| 11 | Glucose | Sigma-Aldrich |
| 12 | LiCl, 8 M | Sigma-Aldrich |
| 13 | CaCl₂, 1 M | Sigma-Aldrich |
| 14 | IP-One - Gq Kit (1,000 tests) | Cisbio |

### 2. Experimental instruments

| No. | Instrument | Source |
|---|---|---|
| 1 | EnVision detector | Perkin Elmer |

### Evaluation of agonist activity of compound of formula (I) on human calcium-sensing receptor (CaSR)

### Experimental procedures:

A stably transfected HEK293/CaSR cell strain (source: Pharmaron) was cultured in a complete medium (composition: DMEM, high glucose + 10% FBS + 2 mM GlutaMAX + 1× Penicillin-Streptomycin + 200 µg/mL Hygromycin B) and incubated at 37 °C/5% CO₂ till 70%-90% cell confluence. The cell strain was digested with TrypLE, inoculated into 384-well cell culture plates, and cultured overnight at 37 °C/5% CO₂. After buffer exchange, stimulation buffer (HEPES 10 mM, MgCl₂ 0.5 mM, KCl 4.2 mM, NaCl 146 mM, glucose 5.5 mM, LiCl 50 mM, CaCl₂ 1.2 mM) and various concentrations of the test example compounds were added and incubated at 37 °C for 60 min. Production of IP-One in cells was detected according to the procedures in the Cisbio IP-One Tb kit instructions. The EC₅₀ values of various test examples in the human calcium-sensing receptor was calculated by software after the raw data of the examples were collected, so as to evaluate the agonist activity of the examples on the human calcium-sensing receptor.

### Data processing:

HTRF signal was read by an EnVision detector with an excitation wavelength of 320 nm and emission wavelengths of 620 nm and 665 nm. The signal ratio (665 nm/620 nm × 10,000) was calculated and fitted non-linearly to the sample concentration in GraphPad Prism 6 using a four-parameter equation to give EC₅₀ values of the test Example 1. The specific values are shown in Table 1 below.

**Table 1: In vitro agonist activity on calcium-sensing receptor**

| Example No. | Sequence | EC₅₀ for calcium-sensing receptor (µM) |
|---|---|---|
| Compound of formula (I) | Ac-c(C)-r-r-(D-Abu)-r-a-r-NH₂ | 6.28 |
| Etelcalcetide | Ac-c(C)-a-r-r-r-a-r-NH₂ | 6.78 |
| Etelcalcetide analogue | Ac-c(C)-r-r-a-r-a-r-NH₂ | 6.74 |

The compound of formula (I) of the present disclosure demonstrated excellent *in vitro* efficacy, corresponding to EC₅₀ values less than 10 µM in the *in vitro* agonist activity evaluation on the human calcium-sensing receptor, comparable to the activity of the positive drug etelcalcetide.

### Evaluation of in vitro activity of compound of formula (I) to induce histamine release in rat peritoneal mast cells

### Procedures and data processing:

To evaluate the *in vitro* histamine release levels induced by some of the test example compounds, rat peritoneal mast cells were collected by lavaging rat peritoneum with a lavage buffer (cold HBSS + 25 mM HEPES containing heparin 5 U/mL, pH 7.4). After collection, the cells were centrifuged, and the lavage buffer was discarded. The cells were resuspended and washed twice with a stimulation buffer (HBSS + 25 mM HEPES + 1 mM CaCl₂, pH 7.4). The cells were plated at a density of 10⁵ cell/well (200 µL/well) and incubated at 37 °C for 15 min with positive control compound 48/80 (final concentration: 4 µg/mL), test example compound (final concentration: 10 µM) or vehicle control. The cells were centrifuged, and cell supernatant was collected and tested for histamine concentration according to LDN Histamine ELISA kit (BAE-1000) instructions. Specific data are shown in Table 2 below.

**Table 2: Histamine release levels in vitro**

| Example No. | Relative fold of histamine release *in vitro* |
|---|---|
| PBS buffer | 1.00 |
| Compound of formula (I) | 0.97 |
| Etelcalcetide | 1.70 |

Etelcalcetide relatively significantly induced histamine release in rat peritoneal mast cells *in vitro,* in particular at a relative histamine release fold higher than 1.50 relative to PBS buffer. Surprisingly, the compound of formula (I) had greatly reduced histamine release levels in rat peritoneal mast cells *in vitro* relative to the etelcalcetide.

### Evaluation of hemolytic effect of compound of formula (I) on human red blood cells in vitro

### Procedures and data processing:

To evaluate the hemolytic effect of the example compounds on red blood cells *in vitro,* human whole blood (100 µL) was taken and mixed with a phosphate buffer. The mixture was centrifuged at 4 °C for 10 min and the supernatant was discarded. The red blood cells were resuspended in PBS buffer (900 µL) and centrifuged at 4 °C for 10 min with the supernatant discarded, and the procedures above were repeated once. The compound of formula (I) was dissolved in 1× PBS buffer to a final concentration of 100 µg/mL. The red blood cells were resuspended in solutions of various test example compounds, a polyethylene glycol octyl phenyl ether-100 solution or PBS buffer, and incubated at 37 °C for 1 h. After incubation, the cells were centrifuged at 4 °C for 10 min and the supernatant (100 µL) was pipetted and transferred to a 96-well plate. The absorbance at 540 nm was detected for evaluating the hemolytic effect of the compound of formula (I) on red blood cells *in vitro.*

### 3.3.3 Results

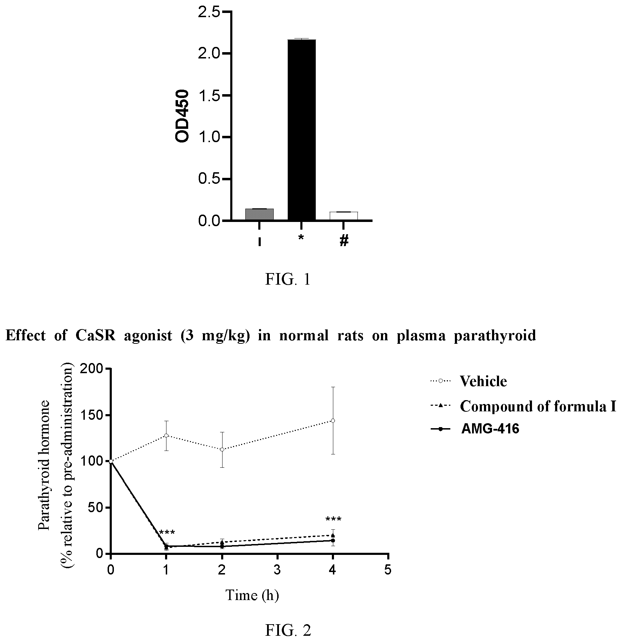

At a concentration of 100 µg/mL, no significant hemolytic effect on red blood cells was observed for the compound of formula (I), while the polyethylene glycol octyl phenyl ether-100 solution demonstrated a significant hemolytic effect on red blood cells under the experimental conditions, as shown in FIG. 1.

### Evaluation of in vivo efficacy of compound of formula (I) in a normal rat model after a single dose

### Procedures and data processing:

SPF normal adult rats (Sprague Dawley, or SD) with weight of 250-350 g were fed with normal diet in an animal room for 7 days. Rats were randomized into groups of 6, half female and half male, and numbered. The day before the start of the experiment, 540 µL of blood was collected from each rat, and the plasma parathyroid hormone levels and the serum calcium ion concentration were measured as control values before administration. The plasma separation method comprises using K2-EDTA as the anticoagulant, collecting blood from the jugular vein, placing on ice after collection, centrifuging the whole blood at 6,800 rpm for 6 min at 2-8 °C, gently taking out the upper layer, i.e., the plasma, and storing at 2-8 °C. The serum separation method comprises collecting blood from the jugular vein, letting the whole blood stand at room temperature for 1 h, centrifuging at room temperature at 3,500 rpm for 10 min, gently taking out the upper layer, i.e., the serum, and storing at room temperature. The animals were fasted overnight with free access to water the day before treatment. The day after blood sampling, the compound of formula (I) and etelcalcetide were dissolved in a phosphate buffered saline (PBS, Gibco). The rats were intravenously administered with the compound of formula (I) or etelcalcetide 3 mg/kg or an equal volume of PBS buffer. Subsequently, blood samples were collected as per the following procedures for measuring the parameters. 100 µL of blood was collected at 1 h, 2 h and 4 h post-dose, and the plasma was separated according to the procedures above. The plasma parathyroid hormone levels were measured using the Rat Intact PTH ELISA Kit (Quidel - Immunotopics, Cat. #: 60-2500; ELISA: Enzyme-linked immunosorbent assay) according to the kit instructions. The detailed procedures are as follows: using the streptavidin-preplated reaction strips provided in the kit, 25 µL of reference standard, control or plasma samples were added to the wells. Biotinylated rat parathyroid hormone antibody and rat parathyroid hormone/HRP binding antibody were mixed at a ratio of 1:1, and 100 µL of the mixed solution was added to each well. The reaction strip was sealed with a sealing film, wrapped with an aluminum foil for storage in dark, and shaken on a horizontal shaker at room temperature for 3 h at a rotation speed of 220 rpm. The solutions in the wells were discarded. 350 µL of cleaning working solution was added to the wells for washing and then discarded; 5 washes were performed with the same procedures. Finally the wells were dried. To each well was added 150 µL of horseradish peroxidase ELISA substrate. The reaction strip was sealed with a sealing film, wrapped with an aluminum foil for storage in dark, and shaken on a horizontal shaker at room temperature for 30 min at a rotation speed of 180-220 rpm. 100 µL of ELISA terminating solution was added to each well and shaken on a horizontal shaker at room temperature for 1 min at a rotation speed of 180-220 rpm. The absorbance at 450 nm in each well was detected within 10 min after the addition of the ELISA terminating solution, while the absorbance at 620 nm was subtracted as background. A mixture of horseradish peroxidase ELISA substrate 150 µL and ELISA terminating solution 100 µL was used as the blank control in the absorbance detection. A standard curve according to the absorbance of the reference standard was plotted, and the actual plasma parathyroid hormone concentration was calculated according to the absorbance of other samples and the standard curve. The determination of the serum calcium ion concentration was conducted according to the procedures of the relevant kit.

### Results

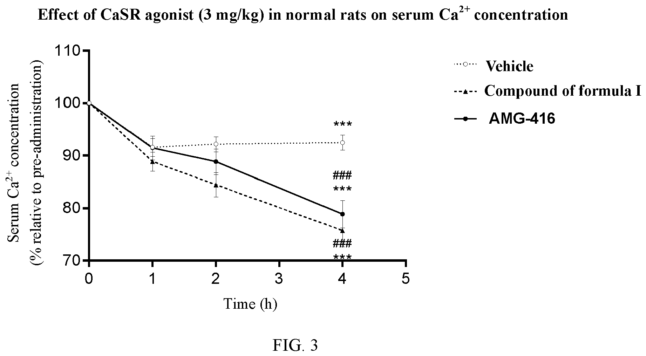

The compound of formula (I) completely reduced the plasma parathyroid hormone levels in normal rats within 4 h at a dose of 3 mg/kg, and the serum calcium ion levels were correspondingly reduced, as shown in FIGs. 2 and 3.

## Claims

1. A pharmaceutical composition comprising an active ingredient, a buffer and an osmotic pressure regulator, wherein the active ingredient is a compound of formula (I) or a pharmaceutically acceptable salt thereof:

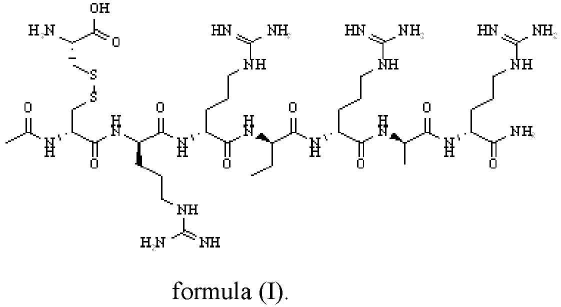

2. The pharmaceutical composition according to claim 1, wherein the composition has a pH value of 2.0-3.5, preferably 3.0-3.5, and more preferably 3.3.

3. The pharmaceutical composition according to claim 1 or 2, wherein the active ingredient is a hydrochloride of the compound of formula (I).

4. The pharmaceutical composition according to any one of claims 1-3, wherein the content of the active ingredient based on free base is 1-10 mg/mL, preferably 2.5-10 mg/mL, and more preferably 5 mg/mL.

5. The pharmaceutical composition according to any one of claims 1-4, wherein the buffer is one or more of succinate, citrate and phosphate, preferably succinate.

6. The pharmaceutical composition according to any one of claims 1-5, wherein the osmotic pressure regulator is one or more of sodium chloride and glucose, preferably sodium chloride.

7. The pharmaceutical composition according to any one of claims 1-6, wherein the buffer has a mass/volume percentage of 0.05%-1%, preferably 0.1%-0.5%, and more preferably 0.118%.

8. The pharmaceutical composition according to any one of claims 1-7, wherein the osmotic pressure regulator has a mass/volume percentage of 0.5%-2%, preferably 0.5%-1%, and more preferably 0.85%.

9. The pharmaceutical composition according to any one of claims 1-8, wherein the composition further comprises a pH adjuster.

10. A pharmaceutical composition comprising 2.5-10 mg/mL of an active ingredient based on free base, 0.1%-0.5% (w/v) of a buffer and 0.5%-1% (w/v) of an osmotic pressure regulator, wherein the active ingredient is a compound of formula (I) or a pharmaceutically acceptable salt thereof, preferably a hydrochloride of the compound of formula (I): 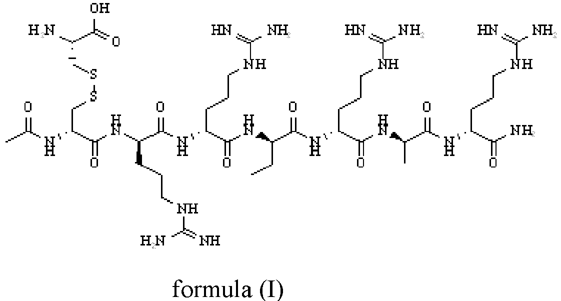
the buffer is one or more of succinate, citrate and phosphate, preferably succinate;
the osmotic pressure regulator is one or more of sodium chloride and glucose, preferably sodium chloride.

11. The pharmaceutical composition according to any one of claims 1-10, wherein the pharmaceutical composition has a total impurity content of 10% or less, preferably 8% or less, when stored at a temperature of 2-8 °C for 3 months.

12. The pharmaceutical composition according to any one of claims 1-11, wherein the pharmaceutical composition is suitable for injection.

13. A method for preparing the pharmaceutical composition according to any one of claims 1-12, comprising the step of mixing the active ingredient, the buffer and the osmotic pressure regulator.

14. Use of the pharmaceutical composition according to any one of claims 1-12 in preparing a medicament for reducing parathyroid hormone levels in a subject or for treating secondary hyperparathyroidism or tumor-induced hypercalcemia.
